# EUROPEAN PATENT APPLICATION

(11) **EP 1 764 686 A1**
(43) Date of publication of application: **21.03.2007**
(21) Application number: 06254802.9
(22) Date of filing: 14.09.2006
(51) Int. Cl.: G06F 9/44

(54) **System and method for dynamic configuration of pacs workstation displays**

(30) Priority: 19.09.2005 US 230169
(71) Applicant: General Electric Company, Schenectady, NY 12345 (US)
(72) Inventor: Vassa, Ashish Dolatrai, Palatine Illinois 60074 (US); Kariathungal, Murali Kumaran, Hoffman Estates Illinois 60194 (US)
(74) Representative: Illingworth-Law, William Illingworth

(57) **Abstract**

Certain embodiments of the present invention provide a method (200) and system (300) for dynamic configuration of PACS workstation (140) displays. Certain embodiments include automatically detecting a number of display(s) (310, 320, 330) in communication with a picture archiving and communication system (PACS) (100) (210) and a display resolution associated with each of the display(s) (310, 320, 330) (220). An application window is created for each of the display(s) (310, 320, 330) such that the application window may be closed, opened and/or resized without affecting other application windows (230). Configuration of display(s) (310, 320, 330) may be dynamically adjusted based upon a number of active displays and a number of applications executing on the PACS (100), for example (240, 260). A display protocol is applied to each application window for display of data in the application window (250). The display protocol may be a default, user-configured and/or dynamically-configured display protocol, for example.

## Description

### BACKGROUND OF THE INVENTION

The present invention generally relates to dynamic display configuration in a picture archiving and communication system. In particular, certain embodiments of the present invention relate to dynamic configuration and management of applications across a plurality of displays in a picture archiving and communication system.

A clinical or healthcare environment is a crowded, demanding environment that would benefit from organization and improved ease of use of imaging systems, data storage systems, and other equipment used in the healthcare environment. A healthcare environment, such as a hospital or clinic, encompasses a large array of professionals, patients, and equipment. Personnel in a healthcare facility must manage a plurality of patients, systems, and tasks to provide quality service to patients. Healthcare personnel may encounter many difficulties or obstacles in their workflow.

A variety of distractions in a clinical environment may frequently interrupt medical personnel or interfere with their job performance. Furthermore, workspaces, such as a radiology workspace, may become cluttered with a variety of monitors, data input devices, data storage devices, and communication device, for example. Cluttered workspaces may result in efficient workflow and service to clients, which may impact a patient's health and safety or result in liability for a healthcare facility. Data entry and access is also complicated in a typical healthcare facility.

Healthcare environments, such as hospitals or clinics, include clinical information systems, such as hospital information systems (HIS) and radiology information systems (RIS), and storage systems, such as picture archiving and communication systems (PACS). Information stored may include patient medical histories, imaging data, test results, diagnosis information, management information, and/or scheduling information, for example. The information may be centrally stored or divided at a plurality of locations. Healthcare practitioners may desire to access patient information or other information at various points in a healthcare workflow. For example, during surgery, medical personnel may access patient information, such as images of a patient's anatomy, that are stored in a medical information system. Alternatively, medical personnel may enter new information, such as history, diagnostic, or treatment information, into a medical information system during an ongoing medical procedure.

A reading, such as a radiology or cardiology procedure reading, is a process of a healthcare practitioner, such as a radiologist or a cardiologist, viewing digital images of a patient. The practitioner performs a diagnosis based on a content of the diagnostic images and reports on results electronically (e.g., using dictation or otherwise) or on paper. The practitioner, such as a radiologist or cardiologist, typically uses other tools to perform diagnosis. Some examples of other tools are prior and related prior (historical) exams and their results, laboratory exams (such as blood work), allergies, pathology results, medication, alerts, document images, and other tools.

Picture archiving and communication systems ("PACS") connect to medical diagnostic imaging devices and employ an acquisition gateway (between the acquisition device and the PACS), storage and archiving units, display workstations, databases, and sophisticated data processors. These components are integrated together by a communication network and data management system. A PACS has, in general, the overall goals of streamlining health-care operations, facilitating distributed remote examination and diagnosis, and improving patient care.

A typical application of a PACS system is to provide one or more medical images for examination by a medical professional. For example, a PACS system can provide a series of x-ray images to a display workstation where the images are displayed for a radiologist to perform a diagnostic examination. Based on the presentation of these images, the radiologist can provide a diagnosis. For example, the radiologist can diagnose a tumor or lesion in x-ray images of a patient's lungs.

Current PACS systems use general techniques known as "hanging protocols" to format display of images. Hanging protocols present a perspective or view to a user, such as a radiologist. Images may be grouped according to characteristics such as DICOM series or series number.

Additionally, PACS systems attempt to prepare images for viewing by users by applying a series of processing steps or functions included in a Default Display Protocol ("DDP"). A DDP is a default workflow that applies a series of image processing functions to image data to prepare the image data for presentation to a user. DDPs typically include processing steps or functions that are applied before any diagnostic examination of the images. A DDP may be based on a type of imaging modality used to obtain the image data, for example. In general, a DDP attempts to present image data in a manner most useful to many users.

With increasing volumes of examinations and images, a reduction of radiologists and mounting pressures on improved productivity, radiologists and other healthcare personnel are in need of image processing or display workflow enhancements that alleviate rote, repetitive tasks. Such enhancements may include the dynamic modification of DDPs so as to incorporate processing functions routinely selected by a radiologist, for example. In other words, improved DDPs that may be modified to incorporate a processing function frequently selected by a given radiologist for a certain type of image, for example, would be highly desirable. The modified DDP may then automatically process subsequent image data according to the processing functions routinely selected by the radiologist. The radiologist would not have to manually select the addition of processing steps as they have been incorporated into the DDP. However, current PACS systems do not provide for such enhancements.

Currently, when a user works on a multiple monitor system, the user may not work on multiple applications if a PACS workflow application, such as Centricity PACS, is running because the PACS workflow application occupies all the monitors and occludes other applications currently displayed on different monitors connected to the system. Such a behavior may be a limiting factor for a user who is working with multiple applications along with a PACS workstation. Additionally, it is highly desirable that none of the PACS features are affected. For example, a user wishes to use a PACS workflow engine, such as Centricity^{™} PACS, with ancillary programs, such as surgical planning tools and/or image processing tools, open. Alternatively, a user wishes to work on Centricity^{™} PACS while referring to a related e-mail in his/her Microsoft Outlook® or other e-mail viewing program, for example. Additionally, a user may want to work on a PACS application while referring to an article on the Internet or other network, for example.

Thus, there is a need for a system and method for dynamic display configuration in a PACS environment. There is a need for a system and method for dynamic configuration and management of applications across a plurality of displays in a PACS environment.

### BRIEF SUMMARY OF THE INVENTION

Certain embodiments of the present invention provide a method and system for dynamic configuration of PACS workstation displays. Certain embodiments provide a method including the steps of automatically detecting a number of one or more displays in communication with a picture archiving and communication system (PACS) and a display resolution associated with each of the one or more displays. The method includes creating an application window for each of the one or more displays such that the application window may be closed and re-opened without affecting other application windows. The method further includes applying a display protocol to each application window for display of data in the application window.

In an embodiment, the detecting step further includes dynamically detecting a change in a number of active displays in communication with the PACS. A configuration of the one or more displays may be dynamically adjusted to create a window for a PACS application spanning all available display area across the one or more displays. In an embodiment, configuration of the one or more displays may be dynamically adjusted based upon a number of active displays and a number of applications executing on the PACS, for example. In an embodiment, the creating step further includes creating multiple application windows for at least one of the display(s). The creating step may also include creating a single application window spanning multiple displays.

The display protocol may be a default and/or user-configured display protocol, for example. The display protocol may be a dynamically configured display protocol, for example. In an embodiment, a different display protocol is applied to each of the display(s) based on the display resolution associated with each of the display(s).

Certain embodiments provide a dynamic windowing system for a picture archiving and communication environment. The system includes a PACS workstation and at least one display in communication with the PACS workstation. The PACS workstation automatically detects a number of display(s) in communication with the PACS workstation and a display resolution associated with each of the display(s). The PACS workstation generates a primary window, transparent to a user, for managing a plurality of application windows across the display(s) such that each of the application windows may be closed, opened and/or resized without affecting other application windows.

In an embodiment, the PACS workstation applies a display protocol to each of the application windows for display of an application and/or data in each of the application windows. In an embodiment, a different display protocol is applied to the display(s) based on the display resolution associated with the display(s). The PACS workstation may dynamically adjust a configuration of the display(s) to create an application window spanning all available display area across the display(s). The PACS workstation may dynamically adjust a configuration of the display(s) based upon a number of active displays and a number of applications executing on the PACS workstation.

Certain embodiments provide a computer-readable storage medium including a set of instructions for execution on a processor. The set of instructions includes a detection routine for automatically detecting a number of displays in communication with a PACS workstation, and a windowing routine for managing a plurality of application windows across a plurality of displays using a primary window that is transparent to a user. The windowing routine dynamically creates the plurality of application windows based on application(s) and/or data to be displayed. In an embodiment, the detection routine further detects a display resolution associated with each display.

The windowing routine may manage the plurality of application windows such that each of the plurality of application windows may be closed, opened and/or resized without affecting an application or data displayed in other windows. The windowing routine may dynamically manage configuration of the plurality of displays based upon a number of active displays and a number of applications executing on the PACS workstation. The windowing routine may apply a display protocol to each of the plurality of application windows for display of an application and/or data in each of the plurality of application windows. In an embodiment, the display protocol is a dynamically configured display protocol, for example.

### BRIEF DESCRIPTION OF SEVERAL VIEWS OF THE DRAWINGS

Embodiments of the present invention will now be described, by way of example only, with reference to the accompanying drawings, in which:
FIG. 1 illustrates an exemplary picture archiving and communication system used in accordance with an embodiment of the present invention.
FIG. 2 illustrates a flow diagram for a method for dynamic display configuration for a PACS workstation used in accordance with an embodiment of the present invention.
FIG. 3 illustrates an example of window management on a three monitor PACS workstation used in accordance with an embodiment of the present invention.
FIG. 4 illustrates an example of window management on a three monitor PACS workstation used in accordance with an embodiment of the present invention.
FIG. 5 illustrates an example of window management on a three monitor PACS workstation used in accordance with an embodiment of the present invention.
FIG. 6 illustrates an example of window management on a three monitor PACS workstation used in accordance with an embodiment of the present invention.
FIG. 7 illustrates an example of window management on a three monitor PACS workstation used in accordance with an embodiment of the present invention.
FIG. 8 illustrates an example of window management on a three monitor PACS workstation used in accordance with an embodiment of the present invention.
FIG. 9 illustrates an example of window management on a three monitor PACS workstation used in accordance with an embodiment of the present invention.
FIG. 10 illustrates an example of window management on a three monitor PACS workstation used in accordance with an embodiment of the present invention.
FIG. 11 illustrates an example of window management on a three monitor PACS workstation used in accordance with an embodiment of the present invention.
The foregoing summary, as well as the following detailed description of certain embodiments of the present invention, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

### DETAILED DESCRIPTION OF THE INVENTION

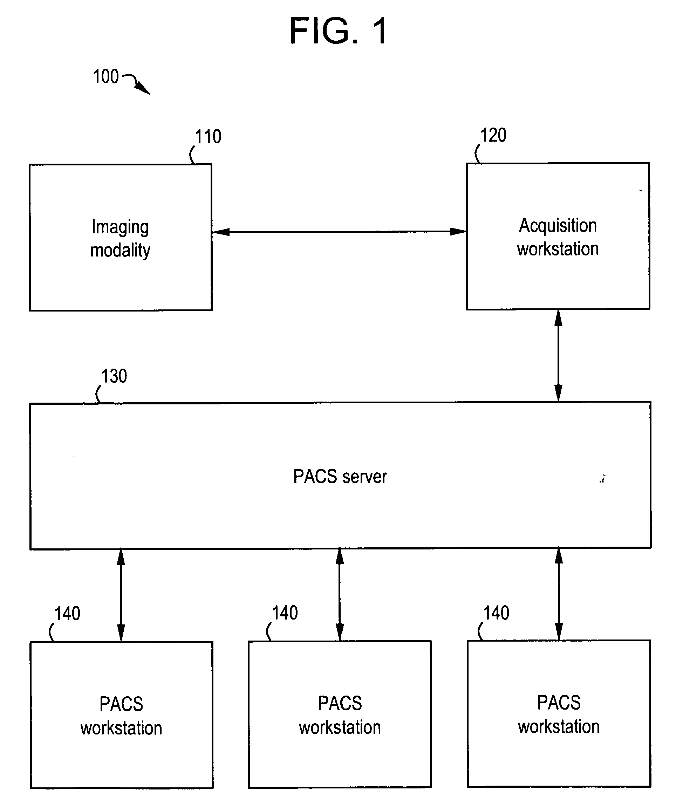

FIG. 1 illustrates an exemplary Picture Archiving and Communication System (PACS) 100 used in accordance with an embodiment of the present invention. The PACS system 100 includes an imaging modality 110, an acquisition workstation 120, a PACS server 130, and one or more PACS workstations 140. The system 100 may include any number of imaging modalities 110, acquisition workstations 120, PACS server 130 and PACS workstations 140 and is not in any way limited to the embodiment of system 100 illustrated in FIG. 1. The components of the system 100 may communicate via wired and/or wireless communication, for example, and may be separate systems and/or integrated to varying degrees, for example.

In operation, the imaging modality 110 obtains one or more images of a patient anatomy. The imaging modality 110 may include any device capable of capturing an image of a patient anatomy such as a medical diagnostic imaging device. For example, the imaging modality 110 may include an X-ray imager, ultrasound scanner, magnetic resonance imager, or the like. Image data representative of the image(s) is communicated between the imaging modality 110 and the acquisition workstation 120. The image data may be communicated electronically over a wired or wireless connection, for example.

In an embodiment, the acquisition workstation 120 may apply one or more preprocessing functions, for example, to the image data in order to prepare the image for viewing on a PACS workstation 140. For example, the acquisition workstation 120 may convert raw image data into a DICOM standard format or attach a DICOM header. Preprocessing functions may be characterized as modality-specific enhancements, for example (e.g., contrast or frequency compensation functions specific to a particular X-ray imaging device), applied at the beginning of an imaging and display workflow. The preprocessing functions differ from processing functions applied to image data in that the processing functions are not modality specific and are instead applied at the end of the imaging and display workflow (for example, at a display workstation 140).

The image data may then be communicated between the acquisition workstation 120 and the PACS server 130. The image data may be communicated electronically over a wired or wireless connection, for example.

The PACS server 130 may include computer-readable storage media suitable for storing the image data for later retrieval and viewing at a PACS workstation 140. The PACS server 130 may also include one or more software applications for additional processing and/or preprocessing of the image data by one or more PACS workstations 140.

One or more PACS workstations 140 are capable of or configured to communicate with the server 130. The PACS workstations 140 may include a general purpose processing circuit, a PACS server 130 interface, a software memory, and/or an image display monitor, for example. The PACS server 130 interface may be implemented as a network card connecting to a TCP/IP based network, but may also be implemented as a parallel port interface, for example.

The PACS workstations 140 may retrieve or receive image data from the server 130 for display to one or more users. For example, a PACS workstation 140 may retrieve or receive image data representative of a computed radiography (CR) image of a patient's chest. A radiologist or user may then examine the image for any objects of interest, such as tumors, lesions, etc., for example.

The PACS workstations 140 may also be capable of or configured to apply processing functions to image data. For example, a user may desire to apply processing functions to enhance features within an image representative of the image data. Processing functions may therefore adjust an image of a patient anatomy in order to ease a user's diagnosis of the image. Such processing functions may include any software-based application that may alter a visual appearance or representation of image data. For example, a processing function can include any one or more of flipping an image, zooming in an image, panning across an image, altering a window and/or level in a grayscale representation of the image data, and altering a contrast and/or brightness an image.

In an embodiment, the PACS system 100 may provide one or more perspectives for viewing images and/or accessing applications at a PACS workstation 140. Perspectives may be provided locally at the PACS workstation 140 and/or remotely from the PACS server 130. In an embodiment, the PACS system 100 includes a perspectives manager capable of being used for reviewing images via a plurality of perspectives. The PACS server 130 and/or a PACS workstation 140 may include the perspectives manager, or the perspectives manager may be implemented in a separate system. In an embodiment, each PACS workstation 140 may include a perspectives manager.

In operation, for example, a user, such as a radiologist, selects a set of images, such as screening mammogram images, chest screening images and/or other computed radiography (CR), digital radiography (DR), and/or digital x-ray (DX) screening images, to review at a PACS workstation 140. The images may be displayed in a default perspective and/or a customized perspective, for example.

As described above, a user may wish to apply additional processing steps to one or more images to further enhance features in the image. For example, a user may desire to apply additional processing functions or steps to an image in order to alter the presentation of an image in conformance with the user's confidence level for making an accurate diagnosis. In other words, different users may desire to apply different or additional processing steps than are included in a default image processing workflow.

The additional image processing step(s) may include any image processing step useful to prepare an image for a diagnostic examination. For example, as described above, an image processing step (as a default image processing step or an additional image processing step) can include flipping an image, zooming in an image, panning across an image, and altering one or more of a window, a level, a brightness and a contrast setting of an image. Image data may be displayed on a PACS workstation 140 using the same and/or different processing, display protocol, and/or perspective as other image(s), for example.

PACS workstations 140 may retrieve or receive image data from server 130 for display to one or more users. For example, a PACS workstation 140 may retrieve or receive image data representative of a computed radiography image of a patient's chest. A radiologist may then examine the image as displayed on a display device for any objects of interest such as, for example, tumors, lesions, etc.

PACS workstations 140 are also capable of or configured to retrieve and/or receive one or more hanging protocols from server 130. For example, a default hanging protocol may be communicated to PACS workstation 140 from server 130. A hanging protocol may be communicated between server 130 and a PACS workstation 140 over a wired or wireless connection, for example.

In general, PACS workstations 140 may present images representative of image data retrieved and/or received from server 130. PACS workstations 140 may present the images according to a hanging protocol. As described above, a hanging protocol is a set of display rules for presenting, formatting and otherwise organizing images on a display device of a PACS workstation 140. A display rule is a convention for presenting one or more images in a particular temporal and/or spatial layout or sequence. For example, a hanging protocol may include a set of computer-readable instructions (or display rules, for example) that direct a computer to display a plurality of images in certain locations on a display device and/or display the plurality of images in a certain sequence or order. In another example, a hanging protocol may include a set of computer-readable instructions that direct a computer to place a plurality of images in multiple screens and/or viewports on a display device. In general, a hanging protocol may be employed to present a plurality of images for a diagnostic examination of a patient anatomy featured in the images.

A hanging protocol may direct, for example, a PACS workstation 140 to display an anterior-posterior ("AP") image adjacent to a lateral image of the same anatomy. In another example, a hanging protocol may direct PACS workstation 140 to display the AP image before displaying the lateral image. In general, a hanging protocol dictates the spatial and/or temporal presentation of a plurality of images at PACS workstation 140.

A hanging protocol differs from a default display protocol ("DDP"). In general, a DDP is a default workflow that applies a series of image processing functions to image data. The image processing functions are applied to the image data in order to present an image (based on the image data) to a user. The image processing functions alter the appearance of image data. For example, an image processing function may alter the contrast level of an image.

DDPs typically include processing steps or functions that are applied before any diagnostic examination of the images. For example, processing functions may be applied to image data in order to enhance features within an image (based on the image data). Such processing functions can include any software-based application that may alter a visual appearance or representation of image data. For example, a processing function can include any one or more of flipping an image, zooming in an image, panning across an image, altering a window and/or level setting in a representation of the image data, and altering a contrast and/or brightness setting in a representation of the image data.

DDPs are usually based on a type of imaging modality used to obtain the image data. For example, image data obtained with a C-arm imaging device in general or a particular C-arm imaging device may have a same or similar DDP applied to the image data. In general, a DDP attempts to present image data in a manner most useful to many users.

Conversely, applying a hanging protocol to image data does not alter the appearance of an image (based on the image data), but instead dictates how the image(s) is (are) presented, as described above.

Server 130 may store a plurality of hanging protocols and/or DDPs. The hanging protocols and/or DDPs that are stored at server 130 and have not yet been modified or customized are default hanging protocols/DDPs. A default hanging protocol and/or DDP may be selected from a plurality of default hanging protocols and/or DDPs based on any number of relevant factors such as, for example, a manual selection, a user identity, and/or pre-processing of the image data.

Specifically, a default hanging protocol and/or DDP may be selected based on a manual selection simply by communicating the default protocol once a user has selected that particular protocol. The user may make the selection, for example, at a PACS workstation 140.

In another example, a default protocol may be selected based on a user identity. For example, a user may have a preferred DDP. The DDP may have been customized to meet the user's preferences for a particular temporal and/or spatial layout of images. Once a user gains access to a PACS workstation 140 (for example, by entering a correct login and password combination or some other type of user identification procedure), the preferred DDP may be communicated to the PACS workstation 140, for example.

In another example, a default protocol may be selected based on pre-processing of image data. Pre-processing of image data may include any image processing known to those of ordinary skill in the art that prepares an image for review by a user. Pre-processing may also include, for example, a computer-aided diagnosis ("CAD") of image data. CAD of image data may include a computer (or similar operating unit) automatically analyzing image data for objects of interest. For example, a CAD may include a software application that analyzes image data for nodules in images of lungs, lesions, tumors, etc. However, a CAD application can include any automatic analysis of image data known to those of ordinary skill in the art.

For example, a default hanging protocol that corresponds to CAD findings of lung tumors may provide for the presentation of the posterior-anterior (PA) and lateral lung images adjacent to each other followed by the presentation of the computer tomography (CT) lung images, followed by the magnetic resonance (MR) lung images, for example. In general, a default hanging protocol that corresponds to CAD findings is designed to present images in a spatial and/or temporal layout that is useful to a radiologist. For example, a radiologist may be greatly assisted in his or her review of the CAD findings by viewing the PA and lateral lung images adjacent to each other, followed by previously acquired multi-slice CT and MR images of the lungs.

Therefore, based on CAD findings, a default protocol may be selected from a plurality of default protocols and applied at a workstation 140 in order to present images to a user.

PACS users often wish to run multiple applications on a PACS workstation 140. In addition to a primary PACS workflow or interface application, a user may wish to access other applications such as surgical planning tools, scheduling tools, electronic mail viewers, image processing tools, and/or other tools. For example, PACS users often like to use a PACS workflow engine while viewing electronic mail and accessing information on the Internet. Users of an integrated RIS/PACS system may wish to access both RIS and PACS applications simultaneously. Typically, however, the PACS application occupies all active display area and hides other applications running on the workstation 140. For example, in a PACS workstation 140 having three monitors, the PACS workflow application occupies all three monitors. When an application is initiated, another application may be displaced, or the application may be launched in a sub-optimal display area. For example, a user may launch a data management or diagnostic processing software at a three-monitor PACS workstation 140, and the application may launch on a color monitor, displacing images displayed on the color monitor. Typically, a user would have to manually reorganize applications to display the management application on a grayscale monitor and the images on the higher resolution color monitor.

Certain embodiments provide an adaptable PACS system 100 accommodating a plurality of displays such that each display operates with a separate display window. All display windows are controlled internally by a primary window that is transparent to users. The primary, transparent window tracks which window or windows have the PACS application and which window(s) have other applications and/or data. Thus, the PACS application and other applications may be simultaneously displayed on a plurality of displays.

Certain embodiments provide dynamic configuration of displays associated with PACS workstation 140. The primary window allows interaction or application(s) and data across multiple windows. The PACS workstation 140 operates a transparent, primary window including a plurality of windows across a plurality of displays.

Additionally, the PACS workstation 140 dynamically adjusts a Default Display Protocol (DDP) for the display(s) based on content and/or connected display(s). For example, if the PACS workstation 140 is reconfigured from a three monitor configuration to a one monitor configuration, the DDP may be modified accordingly. Certain embodiments adapt a DDP based on application(s) closed and/or opened as well as window(s) activated and/or deactivated. For example, a DDP may determine what information is displayed to a user. A DDP may adapt based on a number of available monitors and a number of images to be displayed, for example (e.g., four images are shown on one available display; eight images are shown on two available displays, etc). PACS workstation 140 may configure a DDP for any multi-monitor full screen and/or partial screen applications. Additionally, one or more applications may be resized on a single screen (e.g., minimize, maximize, and/or resize).

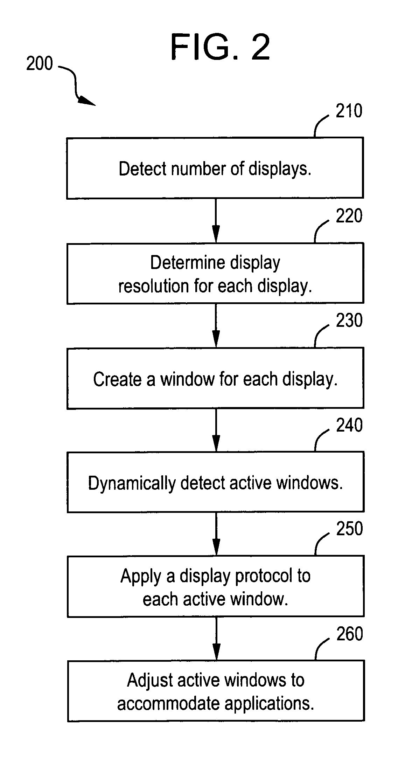

FIG. 2 illustrates a flow diagram for a method 200 for dynamic display configuration for a PACS workstation used in accordance with an embodiment of the present invention. First, at step 210, a number of displays in communication with a PACS workstation 140 is detected. Then, at step 220, a display resolution is determined for each detected display.

At step 230, a window or viewport is created per display such that each window may be closed and re-opened without affecting another application window. In an embodiment, a single window may span multiple displays and/or portions of a display. In an embodiment, multiple windows may be crated for a single display. Next, at step 240, active windows are dynamically detected.

Then, at step 250, a DDP may be applied to the active windows. In an embodiment, one or more DDPs may be applied to one or more active windows. A DDP allows a user to customize image display area on a PACS. A number of active displays may be detected and user-configured DDPs may be applied for all exams or other information displayed, for example. In an embodiment, DDPs may be dynamically configured for information display.

At step 260, active windows are adjusted to accommodate additional applications. In an embodiment, active windows are automatically adjusted to accommodate additional applications without prompting a user. For example, RIS applications, dictation applications, scheduling applications, communication applications, image processing applications, surgical planning applications, and the like may be launched by a user at PACS workstation 140, and active windows are automatically adjusted to accommodate applications in addition to the PACS interface. Applications may occupy a partial display or an entire display when launched. The PACS may adjust itself to the available monitor space that is not occupied by other applications. In an embodiment, a user may manually adjust display allocation.

In an embodiment, a change in a number of active displays may be dynamically detected. Configuration of one or more displays may be dynamically adjusted to create a window for a PACS application spanning all available display area across one or more displays. In an embodiment, configuration of one or more displays may be dynamically adjusted based upon the number of active displays and the number of applications executing on PACS workstation 140, for example. In an embodiment, a different display protocol and/or hanging protocol may be applied to each of the displays based on display resolution or other criteria associated with each of the displays.

The PACS workstation 140 may include computer-readable storage media, such as a hard disk, floppy disk, CD-ROM, DVD, programmable memory, and/or other media, suitable for dynamically managing one or more displays connected to the workstation 140. The PACS workstation 140 may also include one or more software applications for dynamically managing one or more displays connected to the PACS workstation 140. In an embodiment, dynamic window management may be implemented as a set of instructions for execution on a processor, such as a PACS workstation processor. For example, the set of instructions may include a detection routine configured to automatically detect a number of displays in communication with the PACS workstation 140. The set of instructions may also include a windowing routine configured to manage a plurality of application windows across multiple displays using a primary window that is transparent to a user. The windowing routine dynamically creates the plurality of application windows based on applications and data (e.g., image data and/or report data) to be displayed. The windowing routine manages the primary and secondary windows such that the secondary windows may be opened, closed, resized, etc. without affecting applications running in those windows. Display configuration may be dynamically managed based on a number of active displays in communication with the PACS workstation 140 and/or a number of applications executing on PACS workstation 140. The detection routine may detect a display resolution associated with each display. The windowing routine may apply a display protocol to each of the application windows based on display resolution and/or content to be displayed. The display protocol may be a default or user-defined protocol. In an embodiment, the display protocol is a dynamically configured display protocol.

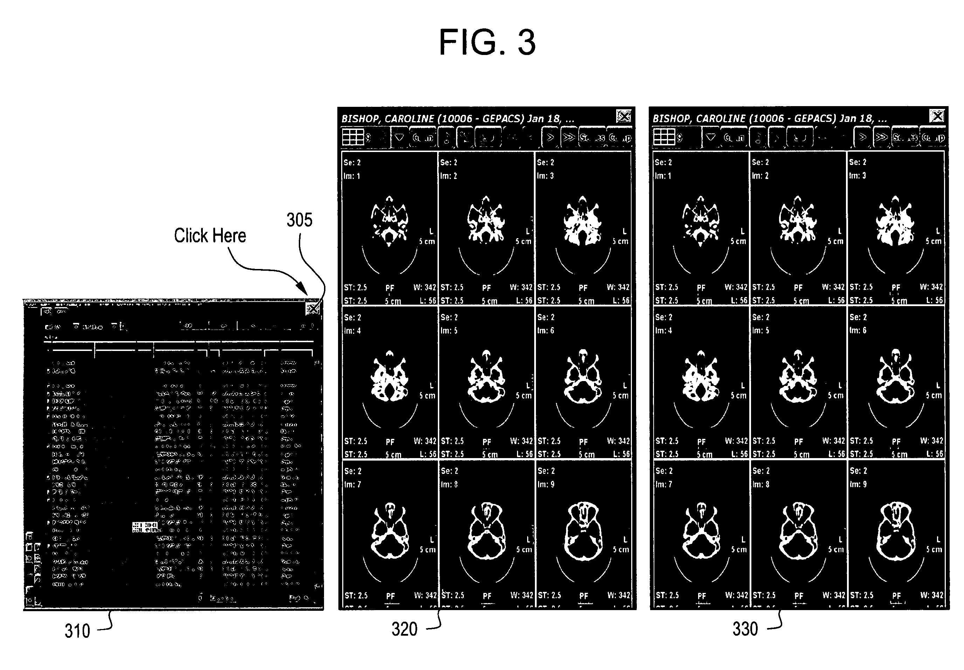

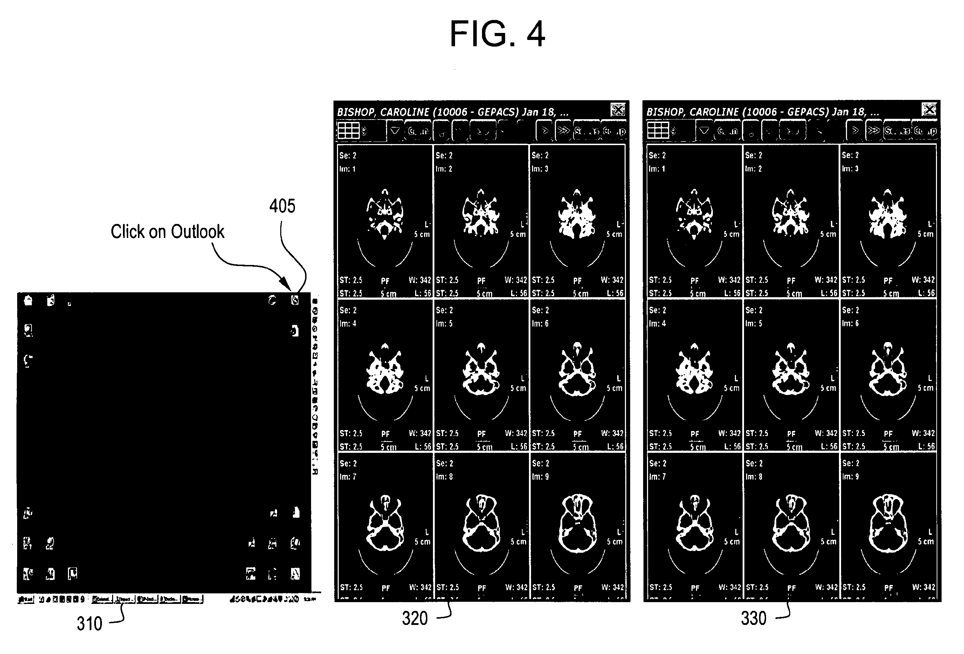

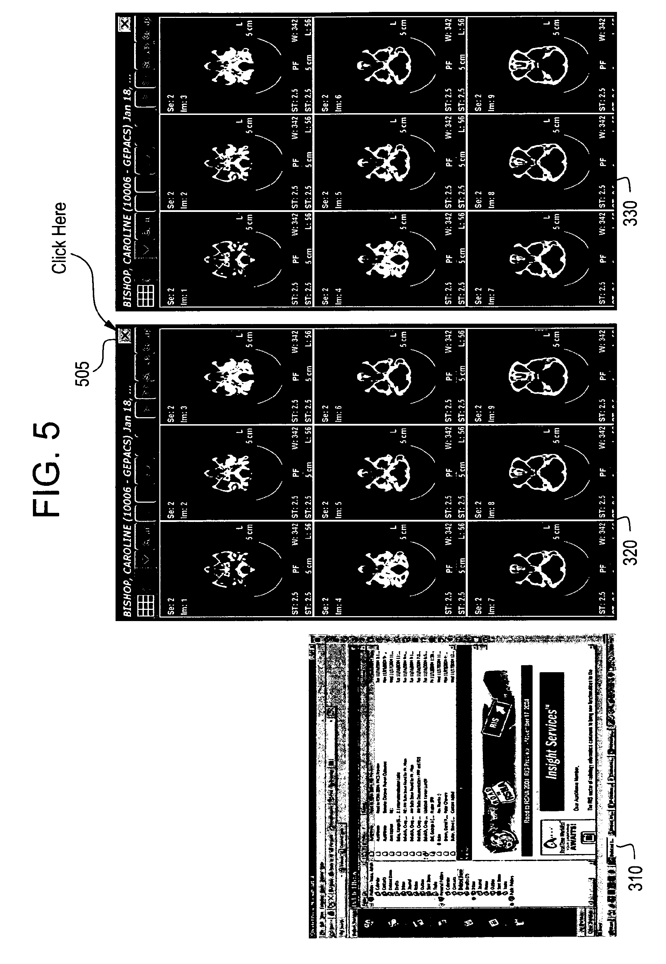

As an example, FIGS. 3-11 illustrate window management on a three display PACS workstation used in accordance with an embodiment of the present invention. FIG. 3 illustrates three screens or displays 310, 320, 330 in communication with a PACS workstation, such as PACS workstation 140. FIG. 3 illustrates a patient listing displayed on color monitor 310 and a series of head CT images displayed on grayscale monitors 320 and 330 corresponding to the patient selected on monitor 310. If a user clicks on or otherwise selects close button 305, the listing closes and reveals the PACS desktop workspace, as shown in FIG. 4. In FIG. 4, a user may select an application, such as Microsoft Outlook, from the desktop. By clicking on or otherwise selecting an icon 405, the application opens in display 310, as shown in FIG. 5.

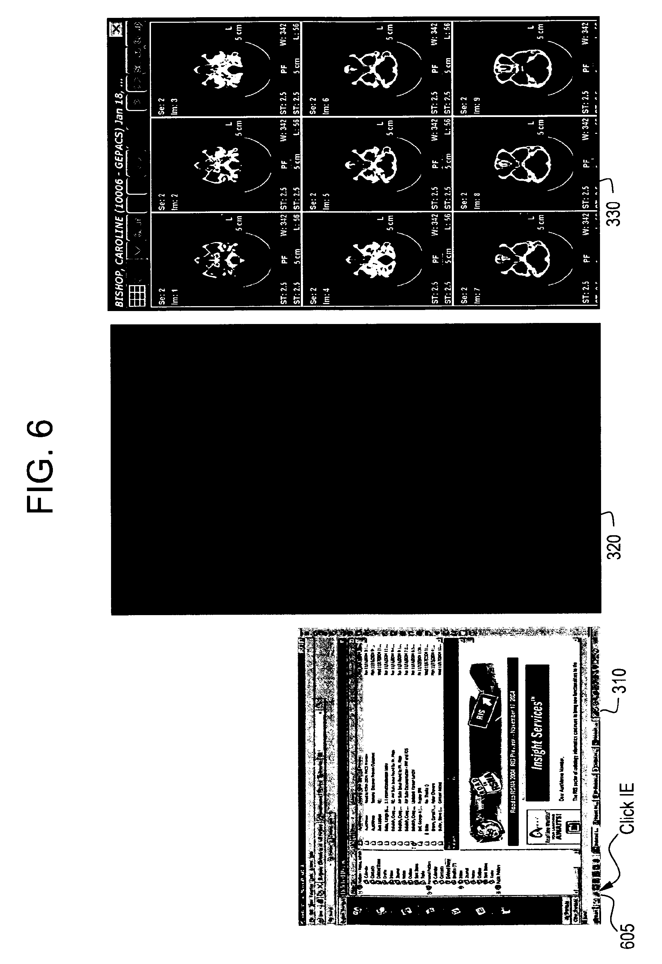

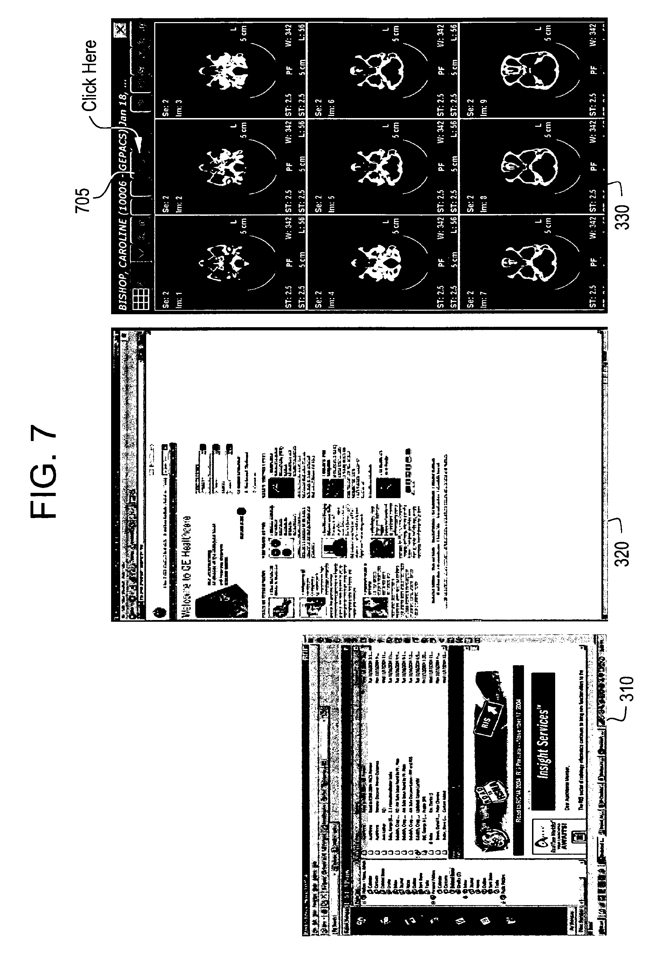

As shown in FIG. 5, a user may close a window displaying CT images at 505 on display 320. In FIG. 6, display 320 is empty, as the image viewing application has been closed for that window. However, a CT image viewing application is still displayed in display 330. A user may select another application icon on the desktop displayed in display 310, such as a Microsoft Internet Explorer icon 605. The window management system detects that display 320 is unused, and shows the new application on display 320, as shown in FIG. 7.

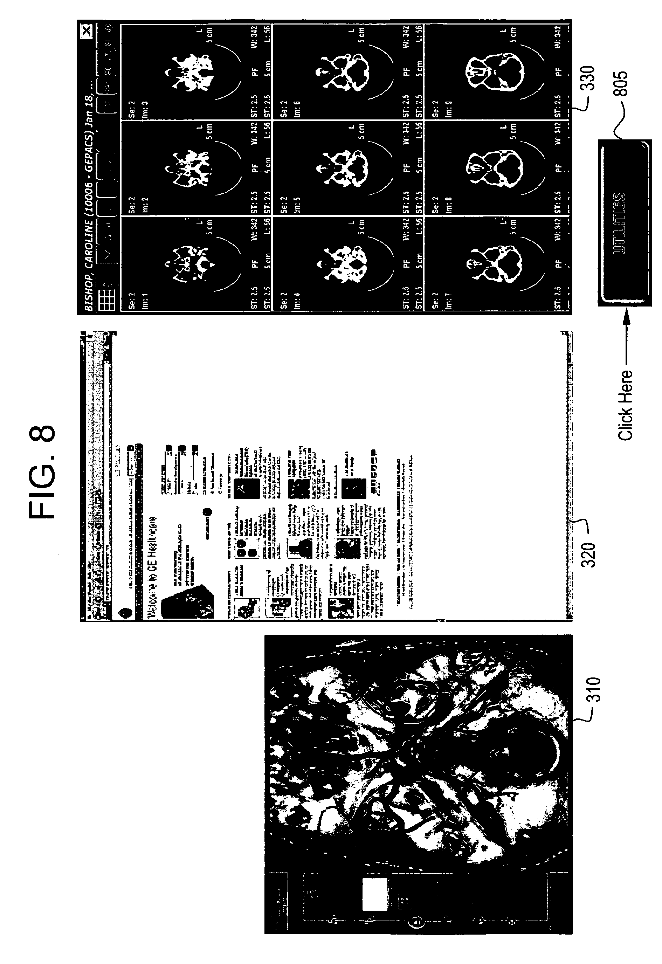

In FIG. 7, a user may select a function, such as a three dimensional rendering function 705, from the image viewing application shown in display 330. FIG. 8 illustrates that the three dimensional rendering has pre-empted the PACS desktop in display 310, the color display. Thus, a color three dimensional rendering is shown on display 310.

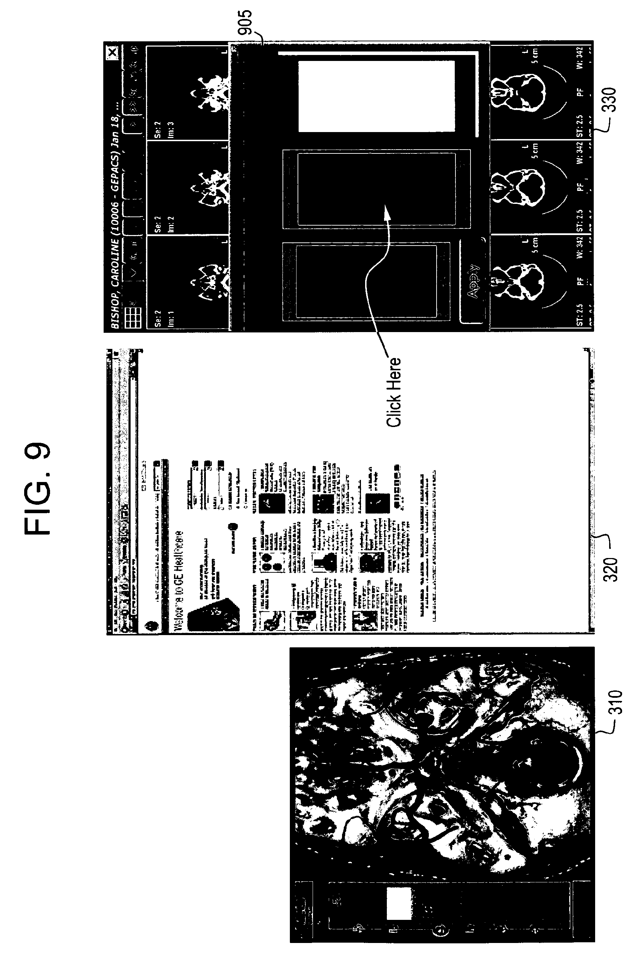

As shown in FIG. 8, utilities or other features may be made available to users via one or more windows/displays on a PACS workstation. Selecting utilities 805 may generate utility options 905 overlaying image data shown in display 330 in FIG. 9.

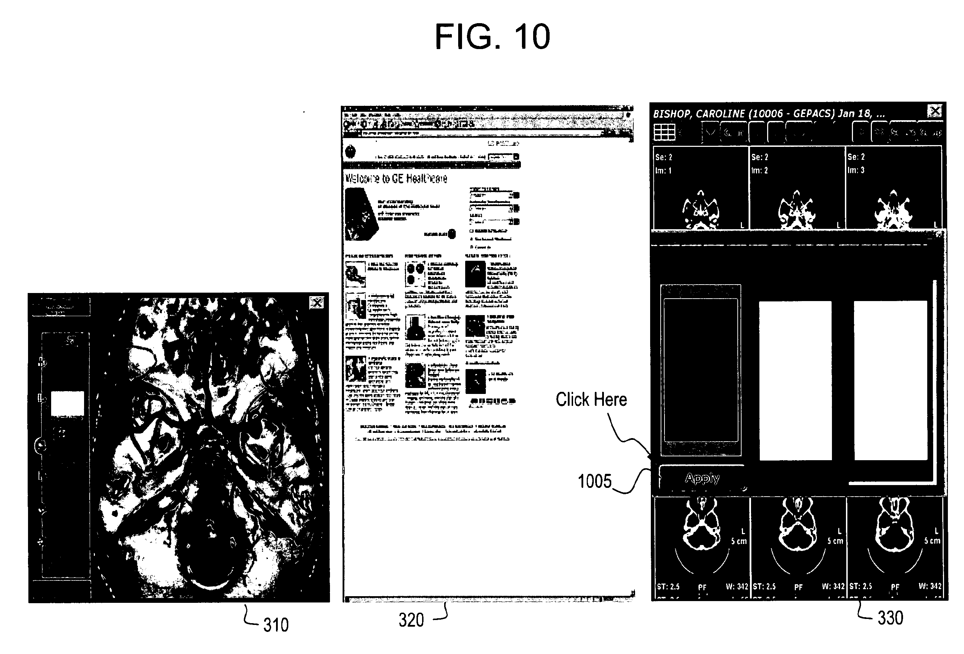

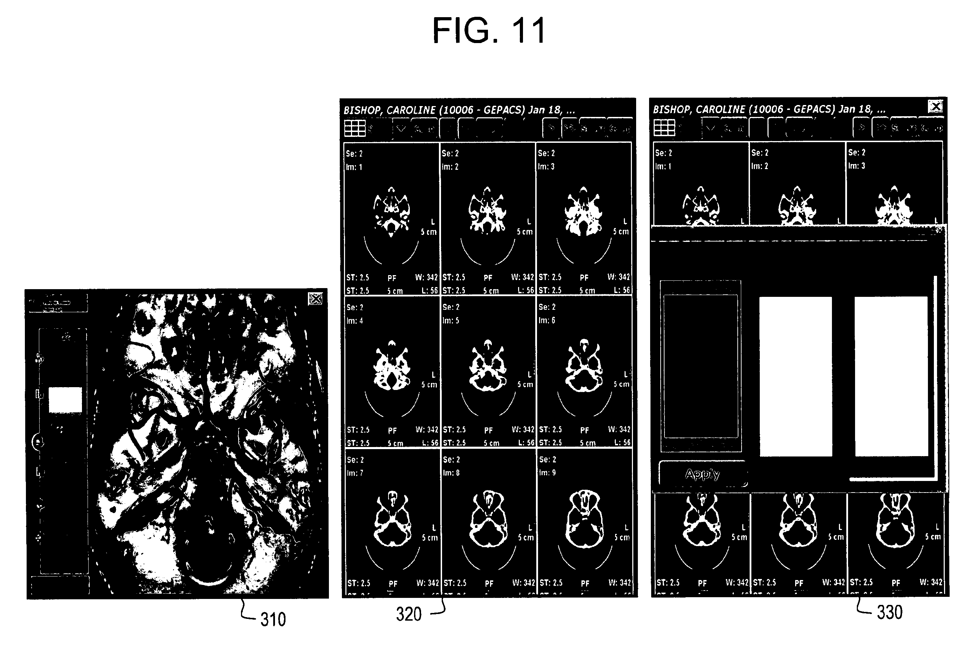

The utility options overlay depicts the three displays 310, 320, 330 in communication with the PACS workstation. A user may select, for example, the center display 320 on the overlay 1005 shown in FIG. 10. Then, as shown in FIG. 11, the PACS viewing application is displayed on the middle display, display 320. That is, a PACS application may be closed or removed from display 320 such as by clicking on the "X" button or close menu option. Additionally, the PACS application may be re-opened or brought back using a utilities menu or other activate option. Thus, certain embodiments facilitate dynamically manipulation of information and configuration of displays in a PACS environment.

Certain embodiments provide shared use of display area with multiple applications. Certain embodiments allow a user to view multiple applications without compromising PACS workstation features. Certain embodiments provide better workflow handling with third party application integration. Certain embodiments provide improved flexibility and monitor configuration. Certain embodiments provide a technical effect of dynamic window management among one or more displays in a PACS, for example.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. Therefore, it is intended that the invention not be limited to the particular embodiment disclosed, but that the invention will include all embodiments falling within the scope of the appended claims.

**PARTS LIST**

| | |
|---|---|
| PACS | 100 |
| Imaging Modality | 110 |
| Acquisition Workstation | 120 |
| PACS Server | 130 |
| PACS Workstation | 140 |
| Flow Diagram | Fig. 2 |
| Close Button | 305 |
| Display | 310 |
| Display | 320 |
| Display | 330 |
| Icon | 405 |
| Close Button | 505 |
| Icon | 605 |
| Rendering Function | 705 |
| Utilities | 805 |
| Utility Options | 905 |
| Overlay | 1005 |

## Claims

1. A method (200) for dynamic configuration of PACS workstation (140) displays, said method (200) comprising:
automatically detecting a number of one or more displays (310, 320, 330) in communication with a picture archiving and communication system (PACS) (100) (210) and a display resolution associated with each of said one or more displays (310, 320, 330) (220);
creating an application window for each of said one or more displays (310, 320, 330) such that said application window may be closed and opened without affecting other application windows (230); and
applying a display protocol to each application window for display of data in the application window (250).

2. The method (200) of claim 1, further comprising dynamically adjusting a configuration of said one or more displays (310, 320, 330) based upon a number of active displays and a number of applications executing on said PACS (100) (240, 260).

3. The method (200) of claim 1, wherein said creating step further comprises creating multiple application windows for at least one of said one or more displays (310, 320, 330).

4. The method (200) of claim 1, wherein said display protocol comprises a user-configured display protocol.

5. The method (200) of claim 1, wherein said display protocol comprises a dynamically configured display protocol.

6. The method (200) of claim 1, wherein a different display protocol is applied to each of said one or more displays (310, 320, 330) based on said display resolution associated with each of said one or more displays (310, 320, 330).

7. A dynamic windowing system (300) for a picture archiving and communication environment, said system (300) comprising:
a picture archiving and communication system (PACS) workstation (140);
at least one display (310, 320, 330) in communication with said PACS workstation (140);
wherein said PACS workstation (140) automatically detects a number of said at least one display (310, 320, 330) in communication with said PACS workstation (140) and a display resolution associated with each of said at least one display (310, 320, 330); and
wherein said PACS workstation (140) generates a primary window, transparent to a user, for managing a plurality of application windows across said at least one display (310, 320, 330) such that each of said plurality of application windows may be closed and opened without affecting other application windows.

8. The system (300) of claim 7, wherein said PACS workstation (140) applies a display protocol to each of said plurality of application windows for display of at least one of an application and data in each of said plurality of application windows.

9. The system (300) of claim 8, wherein a different display protocol is applied to said at least one display (310, 320, 330) based on said display resolution associated with said at least one display (310, 320, 330).

10. The system (300) of claim 7, wherein said PACS workstation (140) dynamically adjusts a configuration of said at least one display (310, 320, 330) based upon a number of active displays and a number of applications executing on said PACS workstation (140).
